# EUROPEAN PATENT SPECIFICATION

(11) **EP 4 389 195 B1**
(45) Date of publication and mention of the grant of the patent: **22.04.2026**
(21) Application number: 21956455.6
(22) Date of filing: 14.09.2021
(51) Int. Cl.: A61N 1/36, A61B 5/053, A61B 5/291, A61B 5/383, A61B 5/0538, A61B 5/00

(54) **SYSTEM FOR ELECTRICAL ACTIVITY SENSING**
SYSTEM ZUR MESSUNG ELEKTRISCHER AKTIVITÄT
SYSTÈME DE DÉTECTION D'ACTIVITÉ ÉLECTRIQUE

(30) Priority: 09.09.2021 CN 202111054819
(43) Date of publication of application: 26.06.2024
(73) Proprietor: SceneRay Co., Ltd., Jiangsu 215000 (CN)
(72) Inventor: LIU, Bin, Suzhou, Jiangsu 215000 (CN); ZHU, Weiran, Suzhou, Jiangsu 215000 (CN); CHEN, Jinghua, Suzhou, Jiangsu 215000 (CN)
(74) Representative: Cabinet Beaumont
(86) International application number: PCT/CN2021/118191
(87) International publication number: WO 2023/035278

(56) References cited:
- WO-A2-2018/008034
- CN-A- 103 108 671
- CN-A- 108 883 275
- CN-A- 110 731 775
- CN-A- 111 886 047
- US-A1- 2011 264 171
- US-A1- 2012 053 658
- US-A1- 2013 218 232
- US-A1- 2018 326 217
- US-A1- 2020 185 093
- CLEMENS M ZIERHOFER* ET AL: "Simultaneous Intracochlear Stimulation Based on Channel Interaction Compensation: Analysis and First Results", IEEE TRANSACTIONS ON BIOMEDICAL ENGINEERING, IEEE, USA, vol. 55, no. 7, 7 July 2008 (2008-07-07), pages 1907 - 1916, XP011205375, ISSN: 0018-9294

## Description

### TECHNICAL FIELD

The present application relates to the technical field of implantable medical systems, and in particular, to a system for an electrical activity sensing, especially, a system for performing an electrical activity sensing while delivering a treatment.

### BACKGROUND

An implantable medical system typically includes an implantable electrical nerve stimulation system, an implantable electrical cardiac stimulation system, and an implantable drug infusion system.

The implantable electrical nerve stimulation system is used as an example, this system mainly includes a pulse generator implanted into a body, an electrode, and a control device outside the body. The pulse generator is connected to the electrode so as to transmit a pulse generated by the pulse generator to the electrode, and a pulse signal generated by the pulse generator is transmitted to a specific nerve part by the electrode to perform the electric stimulation, so that the function of the human body is restored to the normal operation state.

Currently, an electrical signal of deep brain of a patient is collected in a case where the patient does not receive a stimulus, whereby the collected electrical signal cannot reflect a brain condition of the patient during the stimulus.

Further relevant technologies are also known from US 2018/326217 A1 (GIFTAKIS JONATHON E [US] ET AL) 15 November 2018 (2018-11-15) which relates to BRAIN STIMULATION THERAPY.

### SUMMARY

The present invention is set out in the appended set of claims.

### BRIEF DESCRIPTION OF DRAWINGS

Further features, properties and various advantages of the subject matter disclosed in the present application will become more apparent from the following specific implementation modes and the accompanying drawings, in which,

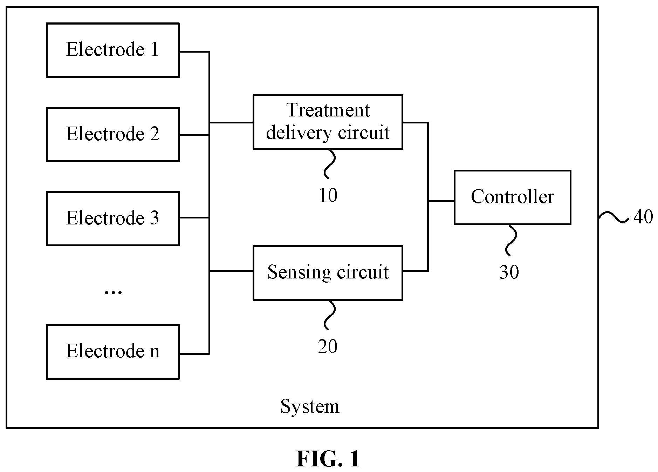

FIG. 1 is a schematic diagram of a simplified block diagram of a system according to an embodiment of the present application.

### DETAILED DESCRIPTION

The present application is further described in conjunction with the accompanying drawings and the specific implementation modes. It should be noted that, in the case of no conflict, the following described embodiments or technical features may be randomly combined to form a new embodiment.

Referring to FIG. 1, an embodiment of the present application provides a system 40. The system includes multiple electrodes, a treatment delivery circuit 10, a sensing circuit 20 and a controller 30. The multiple electrodes are capable of being positioned within a brain of a patient to deliver a treatment to the patient or sense an electrical activity. The treatment delivery circuit 10 is operably coupled to the multiple electrodes to deliver the treatment to the patient. The sensing circuit 20 is operably coupled to the multiple electrodes to sense the electrical activity. The controller 30 includes a processing circuit system operably coupled to the treatment delivery circuit 10 and the sensing circuit 20. The controller 30 is configured to: control, through the treatment delivery circuit 10, one or more electrodes among the multiple electrodes to deliver the treatment to the patient; sense, through the sensing circuit 20, potentials of the multiple electrode in a process of delivering the treatment; and calculate, based on the potentials of the multiple electrodes, a difference value between potentials of any two electrodes among the multiple electrodes to obtain a voltage between the any two electrodes.

The treatment delivery circuit 10 may include a pulse generator and an extension wire connected to the pulse generator, one end of the extension wire is connected to the pulse generator, and another end of the extension wire is connected to the electrode, so as to transmit the pulse generated by the pulse generator to the electrode, and deliver the treatment to the patient by using the electrode. The pulse generator may be coupled to the controller 30.

The controller 30 may include a memory and a processor. A size, a shape, and a position of the controller 30 are not limited in the present application. The control function of the controller 30 may be implemented by an MPU, an MCU, a DSP, an FPGA, or any combination thereof, and the controller 30 may support the wireless upgrading of the embedded program in an integrated chip manner.

According to the system 40 of the present application, when the electrical activity is sensed, one or more electrodes among the multiple electrodes are controlled through the treatment delivery circuit 10 to deliver the treatment to the patient. Moreover, the potentials of the multiple electrodes are sensed through the sensing circuit 20, and the difference value between the potentials of the any two electrodes among the multiple electrodes are calculated based on the potentials of the multiple electrodes to obtain the voltage between the any two electrodes.

Since the electrical activity sensing is performed in synchronization with the delivery treatment of the patient, the measured voltage may reflect, to some extent, a brain condition of the patient when the patient receives the delivery treatment, which has a greater reference value for the subsequent treatment of the patient.

The controller 30 may be further configured to: control, through the treatment delivery circuit 10, one electrode of the multiple electrodes to deliver the treatment to the patient; and acquire, based on voltages between the one electrode and other electrodes of the multiple electrodes, a voltage decay curve in which the one electrode delivers the treatment to the patient, and one or more of impedances of brain tissue between the one electrode and the other electrodes of the multiple electrodes.

Therefore, the voltage decay curve in which the one electrode delivers the treatment to the patient may be acquired, so as to more intuitively reflect the brain condition of the patient when the patient receives the delivery treatment. The one or more of the impedances of the brain tissue between the one electrode and the other electrodes of the multiple electrodes may be acquired, so as to reflect a state of the brain tissue of the patient when the patient receives the delivery treatment to some extent.

In an embodiment, that the voltage decay curve in which the one electrode delivers the treatment to the patient is acquired based on the voltages between the one electrode and the other electrodes of the multiple electrodes includes: the voltage decay curve in which the one electrode delivers the treatment to the patient is plotted by using distances between the one electrode and the other electrodes of the multiple electrodes as a horizontal axis and using voltages between the one electrode and the other electrodes of the multiple electrodes as a vertical axis.

Therefore, the voltage decay curve in which the one electrode delivers the treatment to the patient is plotted by using the distances between the one electrode and the other electrodes of the multiple electrodes as the horizontal axis and using the voltages between the one electrode and the other electrodes of the multiple electrodes as the vertical axis, so that a brain condition of a treatment site in which the one electrode is located and a site corresponding to the other electrodes when the patient receives the delivery treatment may be reflected.

In an embodiment, the controller 30 may be further configured to: determine a first electrode combination including the one electrode based on the voltage decay curve in which the one electrode delivers the treatment to the patient, and the one or more of the impedances of the brain tissue between the one electrode and the other electrodes of the multiple electrodes; and control, through the treatment delivery circuit 10, the first electrode combination to deliver the treatment to the patient.

Therefore, after the voltage decay curve in which the one electrode delivers the treatment to the patient, and the one or more of the impedances of the brain tissue between the one electrode and the other electrodes of the multiple electrodes are obtained, the voltage decay curve and the impedances of the brain tissue may be used as reference, and the first electrode combination is controlled through the treatment delivery circuit 10 to deliver the treatment to the patient.

In an embodiment, the controller 30 may be further configured to: determine a second electrode combination with a slowest decay based on a voltage decay curve in which each electrode of the multiple electrodes delivers the treatment to the patient; and control, through the treatment delivery circuit, the second electrode combination to deliver the treatment to the patient.

Therefore, the second electrode combination with the slowest decay may be determined based on the voltage decay curve in which each electrode of the multiple electrodes delivers the treatment to the patient, and the second electrode combination is controlled through the treatment delivery circuit 10 to deliver the treatment to the patient. Generally, the slower the voltage of the electrode decays, the better the therapeutic effect.

In an embodiment, the electrical activity that is capable of being sensed by the multiple electrodes may include an electrical activity delivering the treatment to the patient and a bioelectrical activity of the patient.

Generally, the signal strength of the electrical activity that delivers the treatment to the patient is larger, and the bioelectrical activity signal of the patient is relatively weak, typically on the order of microvolts.

In an embodiment, the system 40 may further include a filter, and the filter is operably coupled between the sensing circuit 20 and the controller 30 to filter out the bioelectrical activity of the patient sensed by the multiple electrodes or the electrical activity delivering the treatment to the patient sensed by the multiple electrodes.

In one aspect, the filter is provided so that the bioelectrical activity of the patient may be filtered out, whereby the calculated voltage between the electrodes may reflect the strength of the treatment delivered to the patient. On the other hand, the electrical activity that delivers the treatment to the patient may be filtered by using the filter, whereby the calculated voltage between the electrodes may reflect the brain electrical signal strength of the patient.

In an embodiment, the system 40 may further include a signal amplification circuit, and the signal amplification circuit is operably coupled between the sensing circuit 20 and the controller 30 to amplify the bioelectrical activity of the patient sensed by the multiple electrodes. The magnification may be 10 times, 100 times, or 1000 times.

The signal amplification circuit is provided so that the bioelectrical activity of the patient sensed by the multiple electrodes may be amplified for ease of the subsequent calculation.

In an embodiment, the system 40 may further include a signal reduction circuit, and the signal reduction circuit is operably coupled between the sensing circuit 20 and the controller 30 to reduce the electrical activity delivering the treatment to the patient sensed by the multiple electrodes. The reduction multiple may be 2 times, 3 times, or 5 times.

The signal reduction circuit is provided so that the electrical activity delivering the treatment to the patient sensed by the multiple electrodes may be reduced. Generally, an apparatus for measuring the potential of the electrode is relatively precise, and the measurement range is relatively small. The electrical activity delivering the treatment to the patient sensed by the multiple electrodes are reduced, so that the measured potential values may be avoided from exceeding the measurement range.

In an embodiment, the bioelectrical activity of the patient is a single cell electrical activity, a nuclei electrical activity, or a nuclei local electrical activity.

Generally, an electrode required for collecting a single cell electrical signal is different from an electrode required for collecting a local field potential. A single cell requires a small electrode or microelectrode, and the signal collected by the single cell has a high amplitude and a high frequency. The local field potential needs a normal electrode or a macro electrode, the collected signal has a low amplitude and a low frequency, and the size of the normal electrode is, such as, 6 square millimeters.

The system of the present application may sense the single cell electrical activity, the nuclei electrical activity or the nuclei local electrical activity by using the multiple electrodes.

An embodiment of the present application further provides a system. The system includes: a device configured to receive potentials of multiple electrodes in response to determining that one or more electrodes of the multiple electrodes delivers a treatment to a patient; and a device configured to calculate a difference value between potentials of any two electrodes among the multiple electrodes based on the potentials of the multiple electrodes to obtain a voltage between the any two electrodes. The multiple electrodes are capable of being positioned in a brain of the patient to deliver the treatment to the patient or sense an electrical activity.

Thus, the electrical activity may be sensed while the delivery treatment is performed on the patient, and the measured voltage may reflect, to some extent, a brain condition of the patient when the patient receives the delivery treatment, which has a greater reference value for the subsequent treatment of the patient.

In an embodiment, the system may further include: a device configured to control one electrode of the multiple electrodes to deliver the treatment to the patient; and a device configured to calculate, based on voltages between the one electrode and other electrodes of the multiple electrodes, a voltage decay curve in which the one electrode delivers the treatment to the patient, and one or more of impedances of brain tissue between the one electrode and the other electrodes of the multiple electrodes.

Therefore, the voltage decay curve in which the one electrode delivers the treatment to the patient may be acquired, so as to more intuitively reflect the brain condition of the patient when the patient receives the delivery treatment. The impedances of the brain tissue between the one electrode and the other electrodes of the multiple electrodes may be acquired, so as to reflect a state of the brain tissue of the patient when the patient receives the delivery treatment to some extent.

In an embodiment, the device configured to calculate, based on the voltages between the one electrode and the other electrodes of the multiple electrodes, the voltage decay curve in which the one electrode delivers the treatment to the patient, and the one or more of the impedances of the brain tissue between the one electrode and the other electrodes of the multiple electrodes are further configured to: plot the voltage decay curve in which the one electrode delivers the treatment to the patient by using distances between the one electrode and the other electrodes of the multiple electrodes as a horizontal axis and using voltages between the one electrode and the other electrodes of the multiple electrodes as a vertical axis.

Therefore, the voltage decay curve in which the one electrode delivers the treatment to the patient is plotted by using the distances between the one electrode and the other electrodes of the multiple electrodes as the horizontal axis and using the voltages between the one electrode and the other electrodes of the multiple electrodes as the vertical axis, so that a brain condition of a treatment site in which the one electrode is located and a site corresponding to the other electrodes when the patient receives the delivery treatment may be reflected.

In an embodiment, the system may further include: a device configured to determine a first electrode combination including the one electrode based on the voltage decay curve in which the one electrode delivers the treatment to the patient, and the one or more of the impedances of the brain tissue between the one electrode and the other electrodes of the multiple electrodes; and a device configured to control the first electrode combination to deliver the treatment to the patient.

Therefore, after the voltage decay curve in which the one electrode delivers the treatment to the patient, and the one or more of the impedances of the brain tissue between the one electrode and the other electrodes of the multiple electrodes are obtained, the attenuation and the impedances of the brain tissue may be used as reference, and the first electrode combination is controlled to deliver the treatment to the patient

In an embodiment, the system may further include: a device configured to determine a second electrode combination with a slowest decay based on a voltage decay curve in which each electrode of the multiple electrodes delivers the treatment to the patient; and a device configured to control the second electrode combination to deliver the treatment to the patient. Therefore, the second electrode combination with the slowest decay may be determined based on the voltage decay curve in which each electrode of the multiple electrodes delivers the treatment to the patient, and the second electrode combination is controlled to deliver the treatment to the patient. Generally, the slower the voltage of the electrode decays, the better the therapeutic effect.

## Claims

1. A system (40), comprising:
a plurality of electrodes, wherein the plurality of electrodes is capable of being positioned within a brain of a patient to deliver a treatment to the patient or sense an electrical activity;
a treatment delivery circuit (10), wherein the treatment delivery circuit (10) is operably coupled to the plurality of electrodes to deliver the treatment to the patient;
a sensing circuit (20), wherein the sensing circuit (20) is operably coupled to the plurality of electrodes to sense the electrical activity; and
a controller (30), wherein the controller (30) comprises a processing circuit system operably coupled to the treatment delivery circuit (10) and the sensing circuit (20), and the controller is configured to perform: controlling, through the treatment delivery circuit (10), one or more electrodes among the plurality of electrodes to deliver the treatment to the patient; sensing, through the sensing circuit (20), potentials of the plurality of electrodes in a process of delivering the treatment; and calculating, based on the potentials of the plurality of electrodes, a difference value between potentials of two electrodes among the plurality of electrodes to obtain a voltage between the two electrodes;
wherein the system is **characterized in that** the controller (30) is further configured to perform:
controlling, through the treatment delivery circuit (10), one electrode of the plurality of electrodes to deliver the treatment to the patient; and
acquiring, based on voltages between the one electrode and other electrodes of the plurality of electrodes, a voltage decay curve in which the one electrode delivers the treatment to the patient, and acquiring one or more of impedances of brain tissue between the one electrode and the other electrodes of the plurality of electrodes.

2. The system (40) of claim 1, wherein acquiring, based on voltages between the one electrode and other electrodes of the plurality of electrodes, a voltage decay curve in which the one electrode delivers the treatment to the patient comprises: plotting the voltage decay curve in which the one electrode delivers the treatment to the patient by using distances between the one electrode and the other electrodes of the plurality of electrodes as a horizontal axis and using voltages between the one electrode and the other electrodes of the plurality of electrodes as a vertical axis.

3. The system (40) of claim 1, wherein the controller (30) is further configured to perform:
determining a first electrode combination comprising the one electrode based on the voltage decay curve in which the one electrode delivers the treatment to the patient, and the one or more of the impedances of the brain tissue between the one electrode and the other electrodes of the plurality of electrodes; and
controlling, through the treatment delivery circuit (10), the first electrode combination to deliver the treatment to the patient.

4. The system (40) of claim 1, wherein the controller is further configured to perform:
determining a second electrode combination with a slowest decay based on a voltage decay curve in which each electrode of the plurality of electrodes delivers the treatment to the patient; and control, through the treatment delivery circuit (10), the second electrode combination to deliver the treatment to the patient.

5. The system (40) of claims 1 to 4, wherein the electrical activity that is capable of being sensed by the plurality of electrodes comprises an electrical activity delivering the treatment to the patient and a bioelectrical activity of the patient.

6. The system (40) of claim 5, further comprising a filter, wherein the filter is operably coupled between the sensing circuit (20) and the controller (30) to filter out the bioelectrical activity of the patient sensed by the plurality of electrodes or the electrical activity delivering the treatment to the patient sensed by the plurality of electrodes.

7. The system (40) of claim 5, further comprising a signal amplification circuit, wherein the signal amplification circuit is operably coupled between the sensing circuit (20) and the controller (30) to amplify the bioelectrical activity of the patient sensed by the plurality of electrodes.

8. The system (40) of claim 5, further comprising a signal reduction circuit, wherein the signal reduction circuit is operably coupled between the sensing circuit (20) and the controller (30) to reduce the electrical activity delivering the treatment to the patient sensed by the plurality of electrodes.

9. The system of claim 5, wherein the bioelectrical activity of the patient is a single cell electrical activity, a nuclei electrical activity, or a nuclei local electrical activity.

## Patentansprüche

1. System (40), umfassend:
eine Mehrzahl von Elektroden, wobei die Mehrzahl von Elektroden dazu in der Lage ist, innerhalb eines Gehirns eines Patienten positioniert zu werden, um eine Behandlung an den Patienten abzugeben oder eine elektrische Aktivität zu erfassen;
eine Behandlungsabgabeschaltung (10), wobei die Behandlungsabgabeschaltung (10) operativ gekoppelt mit der Mehrzahl von Elektroden ist, um die Behandlung an den Patienten abzugeben;
eine Erfassungsschaltung (20), wobei die Erfassungsschaltung (20) operativ gekoppelt mit der Mehrzahl von Elektroden ist, um die elektrische Aktivität zu erfassen; und
eine Steuereinheit (30), wobei die Steuereinheit (30) ein Verarbeitungsschaltungssystem umfasst, das operativ gekoppelt mit der Behandlungsabgabeschaltung (10) und der Erfassungsschaltung (20) ist, und wobei die Steuereinheit dazu konfiguriert ist, Folgendes durchzuführen: Steuern, durch die Behandlungsabgabeschaltung (10), von einer oder mehreren Elektroden aus der Mehrzahl von Elektroden, um die Behandlung an den Patienten abzugeben; Erfassen, durch die Erfassungsschaltung (20), von Potentialen der Mehrzahl von Elektroden in einem Prozess des Abgebens der Behandlung; und Berechnen, bezogen auf die Potentiale der Mehrzahl von Elektroden, eines Differenzwertes zwischen Potentialen von zwei Elektroden aus der Mehrzahl von Elektroden, um eine Spannung zwischen den zwei Elektroden zu erhalten;
wobei das System **dadurch gekennzeichnet ist, dass** die Steuereinheit (30) ferner dazu konfiguriert ist, Folgendes durchzuführen:
Steuern, durch die Behandlungsabgabeschaltung (10), von einer Elektrode der Mehrzahl von Elektroden, um die Behandlung an den Patienten abzugeben; und
Erhalten, bezogen auf Spannungen zwischen der einen Elektrode und anderen Elektroden der Mehrzahl von Elektroden, einer Spannungsabfallkurve, in welcher die eine Elektrode die Behandlung an den Patienten abgibt, und Erhalten von einer oder mehreren von Impedanzen von Gehirngewebe zwischen der einen Elektrode und den anderen Elektroden der Mehrzahl von Elektroden.

2. System (40) nach Anspruch 1, wobei das Erhalten, bezogen auf Spannungen zwischen der einen Elektrode und anderen Elektroden der Mehrzahl von Elektroden, einer Spannungsabfallkurve, in welcher die eine Elektrode die Behandlung an den Patienten abgibt, Folgendes umfasst: Auftragen der Spannungsabfallkurve, in welcher die eine Elektrode die Behandlung an den Patienten abgibt, unter Verwendung von Abständen zwischen der einen Elektrode und den anderen Elektroden der Mehrzahl von Elektroden als eine horizontale Achse und unter Verwendung von Spannungen zwischen der einen Elektrode und den anderen Elektroden der Mehrzahl von Elektroden als eine vertikale Achse.

3. System (40) nach Anspruch 1, wobei die Steuereinheit (30) ferner dazu konfiguriert ist, Folgendes durchzuführen:
Bestimmen einer ersten Elektrodenkombination, umfassend die eine Elektrode, bezogen auf die Spannungsabfallkurve, in welcher die eine Elektrode die Behandlung an den Patienten abgibt, und die eine oder mehreren von den Impedanzen von Gehirngewebe zwischen der einen Elektrode und den anderen Elektroden der Mehrzahl von Elektroden; und
Steuern, durch die Behandlungsabgabeschaltung (10), der ersten Elektrodenkombination, um die Behandlung an den Patienten abzugeben.

4. System (40) nach Anspruch 1, wobei die Steuereinheit ferner dazu konfiguriert ist, Folgendes durchzuführen:
Bestimmen einer zweiten Elektrodenkombination mit einem langsamsten Abfall bezogen auf eine Spannungsabfallkurve, in welcher jede Elektrode der Mehrzahl von Elektroden die Behandlung an den Patienten abgibt; und Steuern, durch die Behandlungsabgabeschaltung (10), der zweiten Elektrodenkombination, um die Behandlung an den Patienten abzugeben.

5. System (40) nach einem der Ansprüche 1 bis 4, wobei die elektrische Aktivität, die dazu in der Lage ist, durch die Mehrzahl von Elektroden erfasst zu werden, eine elektrische Aktivität, die die Behandlung an den Patienten abgibt, und eine bioelektrische Aktivität des Patienten umfasst.

6. System (40) nach Anspruch 5, ferner umfassend einen Filter, wobei der Filter operativ gekoppelt zwischen der Erfassungsschaltung (20) und der Steuereinheit (30) ist, um die durch die Mehrzahl von Elektroden erfasste bioelektrische Aktivität des Patienten oder die durch die Mehrzahl von Elektroden erfasste elektrische Aktivität, welche die Behandlung an den Patienten abgibt, herauszufiltern.

7. System (40) nach Anspruch 5, ferner umfassend eine Signalverstärkungsschaltung, wobei die Signalverstärkungsschaltung operativ gekoppelt zwischen der Erfassungsschaltung (20) und der Steuereinheit (30) ist, um die durch die Mehrzahl von Elektroden erfasste bioelektrische Aktivität des Patienten zu verstärken.

8. System (40) nach Anspruch 5, ferner umfassend eine Signalreduzierungsschaltung, wobei die Signalreduzierungsschaltung operativ gekoppelt zwischen der Erfassungsschaltung (20) und der Steuereinheit (30) ist, um die durch die Mehrzahl von Elektroden erfasste elektrische Aktivität, welche die Behandlung an den Patienten abgibt, zu reduzieren.

9. System nach Anspruch 5, wobei die bioelektrische Aktivität des Patienten eine elektrische Einzelzellenaktivität, eine elektrische Kernenaktivität oder eine lokale elektrische Kernenaktivität ist.

## Revendications

1. Système (40), comprenant :
une pluralité d'électrodes, dans lequel la pluralité d'électrodes peut être d'être placée à l'intérieur du cerveau d'un patient pour administrer un traitement au patient ou détecter une activité électrique ;
un circuit d'administration de traitement (10), dans lequel le circuit d'administration de traitement (10) est fonctionnellement couplé à la pluralité d'électrodes pour administrer le traitement au patient ;
un circuit de détection (20), dans lequel le circuit de détection (20) est fonctionnellement couplé à la pluralité d'électrodes pour détecter l'activité électrique ; et
un dispositif de commande (30), dans lequel le dispositif de commande (30) comprend un système de circuit de traitement fonctionnellement couplé au circuit d'administration de traitement (10) et au circuit de détection (20), et le dispositif de commande est configuré pour effectuer : la commande, via le circuit d'administration de traitement (10), d'une ou de plusieurs électrodes parmi la pluralité d'électrodes pour administrer le traitement au patient ; détecter, via le circuit de détection (20), des potentiels de la pluralité d'électrodes dans un processus d'administration de traitement ; et calculer, sur la base des potentiels de la pluralité d'électrodes, une valeur de différence entre des potentiels de deux électrodes parmi la pluralité d'électrodes pour obtenir une tension entre les deux électrodes ;
dans lequel le système est **caractérisé en ce que** le dispositif de commande (30) est en outre configuré pour effectuer :
la commande, via le circuit d'administration de traitement (10), d'une électrode de la pluralité d'électrodes pour administrer le traitement au patient ; et
acquérir, sur la base de tensions entre la une électrode et les autres électrodes de la pluralité d'électrodes, une courbe de décroissance de tension dans laquelle la une électrode administre le traitement au patient, et acquérir une ou plusieurs impédances de tissu cérébral entre la une électrode et les autres électrodes de la pluralité d'électrodes.

2. Système (40) selon la revendication 1, dans lequel acquérir, sur la base de tensions entre la une électrode et les autres électrodes de la pluralité d'électrodes, une courbe de décroissance de tension dans laquelle la une électrode administre le traitement au patient comprend : tracer la courbe de décroissance de tension dans laquelle la une électrode administre le traitement au patient en utilisant les distances entre la une électrode et les autres électrodes de la pluralité d'électrodes comme axe horizontal et utiliser les tensions entre la une électrode et les autres électrodes de la pluralité d'électrodes comme axe vertical.

3. Système (40) selon la revendication 1, dans lequel le dispositif de commande (30) est en outre configuré pour effectuer :
déterminer une première combinaison d'électrodes comprenant la une électrode sur la base de la courbe de décroissance de tension dans laquelle la une électrode administre le traitement au patient, et la une ou plusieurs impédances de tissu cérébral entre la une électrode et les autres électrodes de la pluralité d'électrodes ; et
commander, via le circuit d'administration de traitement (10), à la première combinaison d'électrodes d'administrer le traitement au patient.

4. Système (40) selon la revendication 1, dans lequel le dispositif de commande est en outre configuré pour effectuer :
déterminer une deuxième combinaison d'électrodes dotée de la décroissance la plus lente sur la base d'une courbe de décroissance de tension dans laquelle chaque électrode de la pluralité d'électrodes administre le traitement au patient ; et commander, via le circuit d'administration de traitement (10), à la deuxième combinaison d'électrodes d'administrer le traitement au patient.

5. Système (40) selon les revendications 1 à 4, dans lequel l'activité électrique pouvant être détectée par la pluralité d'électrodes comprend l'activité électrique administrant le traitement au patient et l'activité bioélectrique du patient.

6. Système (40) selon la revendication 5, comprenant en outre un filtre, dans lequel le filtre est fonctionnellement couplé au circuit de détection (20) et au dispositif de commande (30) pour filtrer l'activité bioélectrique du patient détectée par la pluralité d'électrodes ou l'activité électrique administrant le traitement au patient détectée par la pluralité d'électrodes.

7. Système (40) selon la revendication 5, comprenant en outre un circuit d'amplification de signal, dans lequel le circuit d'amplification de signal est fonctionnellement couplé entre le circuit de détection (20) et le dispositif de commande (30) pour amplifier l'activité bioélectrique du patient détectée par la pluralité d'électrodes.

8. Système (40) selon la revendication 5, comprenant en outre un circuit de réduction de signal, dans lequel le circuit de réduction de signal est fonctionnellement couplé entre le circuit de détection (20) et le dispositif de commande (30) pour réduire l'activité électrique administrant le traitement au patient détectée par la pluralité d'électrodes.

9. Système selon la revendication 5, dans lequel l'activité bioélectrique du patient est l'activité électrique d'une cellule unique, l'activité électrique des noyaux, ou l'activité électrique locale des noyaux.
